# EUROPEAN PATENT APPLICATION

(11) **EP 1 867 282 A1**
(43) Date of publication of application: **19.12.2007**
(21) Application number: 06730029.3
(22) Date of filing: 27.03.2006
(51) Int. Cl.: A61B 6/00, G03B 42/02

(54) **RADIOGRAPHIC IMAGING SYSTEM**

(30) Priority: 07.04.2005 JP 2005110905; 07.04.2005 JP 2005110919
(71) Applicant: Konica Minolta Medical & Graphic, Inc., Tokyo 163-0512 (JP)
(72) Inventor: Ohara, Hiromu Konica Minolta Medical & Graphic Inc, Hachioji-shi, Tokyo; 192805 (JP)
(74) Representative: Roberts, Mark Peter
(86) International application number: PCT/JP2006/306081
(87) International publication number: WO 2006/109551

(57) **Abstract**

A radiation image radiographing system using a combination of a plurality of consoles and a radiographic image detecting apparatus 6 **characterized by** enhanced image verification efficiency. This radiation image radiographing system includes a radiographic image detecting apparatus 6 for detecting an applied radiation, a plurality of consoles 7 provided with a communication section 19 for communicating with an external apparatus, the consoles 7 being used to operate the radiographic image detecting apparatus 6, a control section 14 as an association device for establishing associations between the radiographic image detecting apparatus 6 and the console 7 for operating the radiographic image detecting apparatus 6 during radiographing operation, and a control section 21 as an image information transmission device for sending the radiographic image information obtained based on the detection result of the radiographic image detecting apparatus 6, to at least the console 7 associated by the control section 14, wherein the console 7 has a display section 17 for displaying the radiographic image information.

## Description

### FIELD OF THE INVENTION

The present invention relates to a radiation image radiographing system, particularly a radiation image radiographing system for capturing a radiographic image using a plurality of consoles and radiographic image detecting apparatus.

### BACKGROUND OF THE INVENTION

In the conventional art of medical diagnosis, a subject is irradiated with radiation such as X rays, and a radiographic image is obtained by detection of the distribution of the intensity of radiation having passed through the subject. This radiographic image has been employed widely in medical diagnosis. In recent years, a radiation image radiographing system has been developed, which uses an FPD (Flat Panel Detector) as a radiographic image detecting apparatus wherein a radiation is detected as radiographic image information at the time of photographing and is converted into an electric signal.

One of these radiation image radiographing systems is linked with a predetermined console such as a PC (Personal Computer) for operating the FPD placed in an X-ray room, via a predetermined communication line for the purpose of enhancing the degree of freedom in system configuration (for example, Patent Document 1).

A proposal has been made of a cassette type FPD intended to enhance the transportability and easy handling of the FPD (for example, Patent Document 2). Further, the cassette type FPD and console are configured so as to permit exchange of various forms of information such as radiographic image data by radio system (for example, Patent Document 3).

In such a radiation image radiographing system as the FPD according to the conventional art, the FPD and console are used in a form associated in a one-to-one correspondence. Normally, the console associated in this manner is used for FPD image processing and others. However, for recent development of the communication network, limited space and other various reasons, one console is often connected with a plurality of FPDs. Further, when there are a plurality of X-ray rooms, a console is installed in each X-ray room, and a plurality of these consoles are linked via a single network. Such a system has been proposed.

In this respect, a proposal has been made of a system using a CR (Computed Radiography), wherein the radiographic images stored on a radiographic image storage sheet is read by a radiographic image reading apparatus so that a digital image is obtained, and wherein a plurality of radiographic image reading apparatuses and console are linked via the network, whereby the image having been read is sent to a predetermined console (for example, Patent Documents 4 and 5).

In the CR, for example, at the time of capturing an image using the radiographic image storage sheet stored in a cassette is used for radiographing, the cassette used for radiographing is registered when the radiographing order information has been associated with the ID for identifying the cassette at the console before photographing. The image having been read is generally sent back to the registered console. For example, one patient is subjected to a plurality of radiographing operations, a plurality of cassettes are registered at one particular console. After radiographing, when the image is read by different radiographic image reading apparatuses such as the ones located in front of respective X-ray rooms, all the image data is concentrated onto the one particular console having been used for the aforementioned registration, and display image such as a thumbnail image can also be shown. This arrangement is conveniently used in a CR system wherein a long reading time is required.
[Patent Document 1] Unexamined Japanese Patent Application Publication No. 2003-199736
[Patent Document 2] Unexamined Japanese Patent Application Publication No. H6-342099
[Patent Document 3] Unexamined Japanese Patent Application Publication No. 2003-210444
[Patent Document 4] Unexamined Japanese Patent Application Publication No. 2002-311524
[Patent Document 5] Unexamined Japanese Patent Application Publication No. 2002-159476

### DISCLOSURE OF INVENTION

### PROBLEMS TO BE SOLVED BY THE INVENTION

Radiographing by a radiographic image detecting apparatus such as an FPD is characterized by very short reading time, as compared to the CR. Thus, the radiation image radiographing system using the radiographic image detecting apparatus such as an FPD permits immediate verification of an image immediately after radiographing operation, and is superior to the system using the CR in points of immediacy and convenience.

At the time of capturing an image, a radiographic image detecting apparatus is commonly operated by the console located closest to the X-ray room wherein the radiographing operation is performed. In the case of a radiographic image detecting apparatus such as a portable cassette type FPD, after a patient to be imaged has been associated with the radiographic image detecting apparatus used for this radiographing operation at a certain console, the radiographic image detecting apparatus having been associated is moved into another X-ray room wherein the radiographing operation is performed, in some cases. In such cases, the console used to operate the radiographic image detecting apparatus at the time of radiographing is different from the console used for associating the patient with the radiographic image detecting apparatus.

Thus, particularly in the system using a radiographic image detecting apparatus such as a portable cassette type FPD, the advantages of immediacy or convenience cannot be sufficiently demonstrated, if all the image data is displayed on a single console used for registration (association), independently of the X-ray room wherein radiographing operation has been performed, similarly to the case of the aforementioned CR system. For example, if the console used for registration is located far away from the console used to operate the radiographic image detecting apparatus at the time of radiographing operation, the image can be verified only after the operator has moved to the console used for registration subsequent to termination of radiographing operation. Thus, the radiographic image detecting apparatus cannot sufficiently demonstrate the advantages of immediately checking the image without requiring much time from radiographing to reading the image. This has been a problem with this method. Further, it is a common practice to use one console to input the patient information. Thus, when the thumbnail image of the captured image on the console used for registration of the patient appears, as disclosed in the Unexamined Japanese Patent Application Publication No. 2002-311524 and Unexamined Japanese Patent Application Publication No. 2002-159476, all the information on the image including the thumbnail image is displayed only on the console used for registration, independently of the X-ray room wherein radiographing operation has been performed. Thus, in this case, it is necessary to go to the console used for the patient registration to check the thumbnail image and others every time, independently of the X-ray room wherein radiographing operation has been performed. This has been a problem with the conventional arrangement.

The radiographing operation system based on FPD does not require much time from radiographing to reading, and allows immediate verification of the image. Thus, in immediacy and convenience, this system is superior to the system using the CR. As described above, if a plurality of pieces of image information obtained from the radiographing operation of a single patient cannot be verified collectively at one position, the advantages of the FPD cannot be sufficiently demonstrated. This problem has been left unsolved.

An object of the present invention is to solve these problems and to provide a radiation image radiographing system using a combination of a plurality of consoles and radiographic image detecting apparatus such as the FPD, wherein the radiology technician work flow is improved and the image verification efficiency is enhanced.

### MEANS FOR SOLVING THE PROBLEMS

To solve the aforementioned problems, the invention of the first claim includes;
a radiographic image detecting apparatus for detecting the radiation having been applied;
a plurality of consoles provided with
a communication section for providing communication with an external apparatus, the aforementioned consoles being used to operate the aforementioned radiographic image detecting apparatus;
an association device for establishing associations between the aforementioned radiographic image detecting apparatus and the aforementioned console for operating the aforementioned radiographic image detecting apparatus at the time of radiographing operation; and
an image information transmission device for sending the radiographic image information obtained based on the result of detection by the aforementioned radiographic image detecting apparatus, to at least the aforementioned console associated by the aforementioned association device;
   wherein the aforementioned console has a display section for displaying the aforementioned radiographic image information.

The invention of the second claim is the radiation image radiographing system described in Claim 1 including a display image generation device for generating a radiographic image for display, based on the detection result of the aforementioned radiographic image detecting apparatus,
wherein the radiographic image information sent to the aforementioned console by the aforementioned image information transmission device is the radiographic image information for display generated by the display image generation device.

The invention of the third claim is the radiation image radiographing system described in Claim 2 wherein the aforementioned radiographic image information for display generated by the aforementioned display image generation device is the reduced-size image information whose amount of information is smaller than that of the original image information.

The invention of the fourth claim is the radiation image radiographing system described in any one of the Claims 1 through 3 wherein the aforementioned radiographic image detecting apparatus is a cassette type flat panel detector (FPD) that detects the radiation having been applied, converts this radiation into an electric signal, stores it, reads the stored electric signal and obtains radiographic image information.

The invention of the fifth claim is the radiation image radiographing system described in Claim 1 further including:
a patient information inputting device for inputting the patient information that identifies the patient as a subject;
a patient information association device that establishes associations between the aforementioned patient information and the result of detection by the aforementioned radiographic image detecting apparatus; and
an image information transmission device that sends the radiographic image information obtained from the aforementioned detection result associated with the patient information, to at least the aforementioned console associated as the one for controlling the aforementioned radiographic image detecting apparatus at the time of radiographing when the last image of the same patient is captured.

The invention of the sixth claim is the radiation image radiographing system described in Claim 5 further including a patient list generating device for creating a patient list based on at least the aforementioned patient information inputted through the aforementioned patient information inputting device, wherein the aforementioned console has a patient selecting device for selecting a patient from the aforementioned_patient list, and the aforementioned patient information association device associates the patient information with the result of detection obtained by this radiographing operation, the patient information being the one for the patient selected by the patient selecting device at the time of radiographing operation.

The invention of the seventh claim is the radiation image radiographing system described in Claim 5 or Claim 6 further including a display image generation device for generating the radiographic image information for display based on the result of detection by the aforementioned radiographic image detecting apparatus;
wherein the radiographic image information sent to the aforementioned console by the aforementioned image information transmission device is a radiographic image information for display generated by the display image generation device.

The invention of the eighth claim is the radiation image radiographing system described in Claim 7 wherein the aforementioned radiographic image information for display generated by the aforementioned display image generation device is the reduced-size image information whose amount of information is smaller than that of the original image information.

The invention of the ninth claim is the radiation image radiographing system described in any one of the Claims 5 through 8 wherein the aforementioned radiographic image detecting apparatus is a cassette type flat panel detector (FPD) that detects the radiation having been applied, converts this radiation into an electric signal, stores it, reads the stored electric signal and obtains radiographic image information.

### EFFECTS OF THE INVENTION

According to the invention described in Claim 1, the console for operating the radiographic image detecting apparatus at the time of radiographing operation is normally positioned close to the X-ray room for this radiographing operation to ensure operation convenience, whereby this X-ray room is managed. To be more specific, association is established between the radiographic image detecting apparatus and patient by in the console positioned close to the X-ray room for this radiographing operation to ensure the easiest operation by the radiology technician in charge of radiographing operation, and radiographic image information is provided by the console having established this association. Further, the radiographic image information sent to the console is displayed on the display section of the console. This allows the radiology technician to determine suitability of radiographing operation and need of repeating the radiographing operation immediately after the first radiographing operation, with the result that image verification efficiency is enhanced. This has the effect of sufficiently demonstrating the advantages of the radiographic image detecting apparatus that does not require time from radiographing to reading.

According to the invention described in Claim 2, the radiographic image information for display can be sent to the console. By checking the radiographic image information for display appearing on the display section of the console, the radiology technician can determine suitability of radiographing operation, and the need of repeating the radiographing operation immediately after the first radiographing operation. Thus, image verification efficiency is enhanced.

According to the invention described in Claim 3, the radiographic image information for display is the reduced size image information whose information amount is smaller than that of the original image information. The sending time of the radiographic image information for display is reduced by sending only such reduced-size image information to the console, and quick display of the radiographic image information on the console can be achieved after radiographic imaging. This allows the radiology technician to determine suitability of radiographing operation and the need of repeating the radiographing operation immediately after the first radiographing operation. Thus, image verification efficiency is enhanced.

According to the invention described in Claim 4, a cassette type FPD is used as the radiographic image detecting apparatus. This arrangement provides the advantage of excellent transportability independently of the place where radiographing operation is performed.

According to the invention described in Claim 5, the radiographic image information obtained from the result of detection associated with the patient information is sent to the console associated with the aforementioned radiographic image detecting apparatus that has captured the image of the aforementioned patient last. For example, when radiographing operation has been repeated several times for one patient by moving between X-ray rooms, the radiographic image information for all the radiographing operations can be obtained at one console which is located closest to the X-ray room wherein the last radiographing operation has been performed. The radiographic image information sent to the console is displayed on the display section of the console. Accordingly, even when a plurality of consoles are arranged through one network and radiographing operation has been repeated several times for one patient by moving between X-ray rooms. This allows the radiology technician to determine suitability of radiographing operation and need of repeating the radiographing operation, without moving away from the console wherein the aforementioned last radiographing operation has been performed, with the result that image verification efficiency is enhanced.

According to the invention described in Claim 6, selecting a patent from the patient list makes it possible to get the radiographic image information based on the result of detection associated with the patient information, using the console wherein the patient has been selected. The radiographic image information sent to the console is displayed on the display section of the console. Accordingly, even when a plurality of consoles are arranged through one network and radiographing operation has been repeated several times for one patient by moving between X-ray rooms, the radiology technician can determine suitability of all radiographing operations for this particular patient and the need of repeating the radiographing operations, using a simple means. This arrangement leads to enhanced image verification efficiency.

According to the invention described in Claim 7, radiographic image information for display is sent to the console. Accordingly, by checking the radiographic image information for display appearing on the display section of the console, the radiology technician can determine suitability of radiographing operation, and the need of repeating the radiographing operation immediately after the first radiographing operation. Thus, image verification efficiency is enhanced.

According to the invention described in Claim 8, the radiographic image information for display is the reduced-size image information whose information amount is smaller than that of the original image information. The time for sending the radiographic image information for display is cut down by sending only such reduced-size image information to the console, and quick display of the radiographic image information on the console can be achieved after radiographic imaging. This allows the radiology technician to determine suitability of radiographing operation and the need of repeating the radiographing operation immediately after the first radiographing operation. Thus, image verification efficiency is enhanced.

According to the invention described in Claim 9, a cassette type FPD is used as the FPD. This arrangement provides the advantage of excellent transportability independently of the place where radiographing operation is performed.

### BRIEF DESCRIPTION OF THE DRAWINGS

Fig. 1 is a diagram representing the schematic structure illustrating an embodiment of the radiation image radiographing system of the present invention;
Fig. 2 is a block diagram representing the major structure of the server constituting the radiation image radiographing system of Fig. 1;
Fig. 3 is a block diagram representing the major structure of the console constituting the radiation image radiographing system of Fig. 1;
Fig. 4 is a block diagram representing the major structure of the radiographic image detecting apparatus constituting the radiation image radiographing system of Fig. 1;
Fig. 5 is a flow chart representing the flow of processing at the time of installing a new radiographic image detecting apparatus in an X-ray room;
Fig. 6 is a flow chart representing the flow of processing in the first embodiment of operation at the time of radiographing operation of the radiation image radiographing system;
Fig. 7 is a flow chart representing the flow of processing in the second embodiment of operation at the time of radiographing operation of the radiation image radiographing system;
Fig. 8 is a flow chart representing the flow of processing in the third embodiment of operation at the time of radiographing operation of the radiation image radiographing system; and
Fig. 9 is a flow chart representing the flow of image transfer processing in the third embodiment of operation at the time of radiographing operation of the radiation image radiographing system.

### DESCRIPTION OF REFERENCE NUMERALS

- 1: Radiation image radiographing system
- 2: Server
- 3: Radiation image radiographing apparatus
- 4: Radiation irradiation operation apparatus
- 6: Radiographic image detecting apparatus
- 7: Console
- 18: Input operation section
- 19: Communication section
- 21: Control section
- 25: Image memory
- 26: Communication section
- 28: Input operation section

### BEST FORM OF EMBODIMENT OF THE PRESENT INVENTION

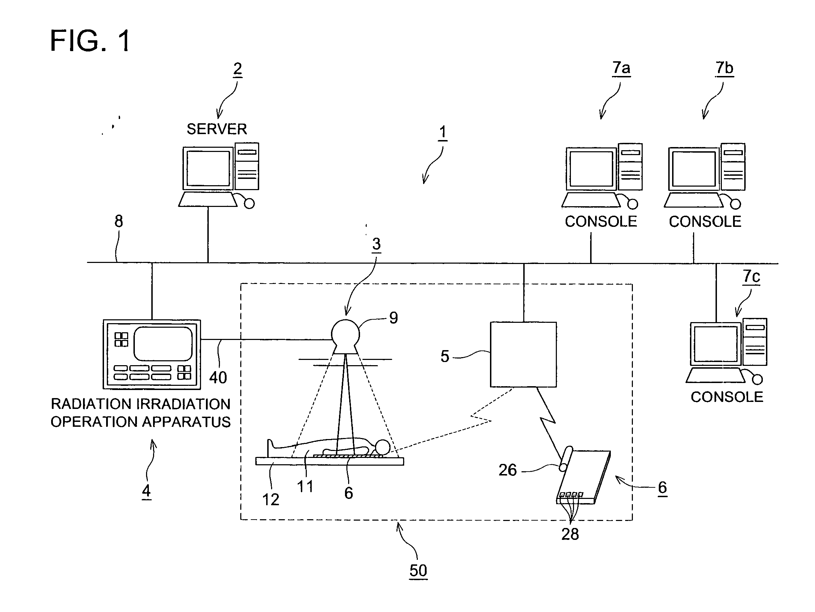

The following describes the embodiments of the present invention with reference to Fig. 1 through Fig. 5:

### [EMBODIMENT 1]

The following describes the first embodiment of the present invention with reference to Fig. 1 through Fig. 4:
Fig. 1 is a diagram representing the schematic structure illustrating an embodiment of the radiation image radiographing system 1 applied with the radiographic image detecting apparatus of the present invention.

The radiation image radiographing system 1 of the present embodiment uses radiation such as X-rays to perform an radiographing operation. As shown in Fig. 1, a server 2 for managing the information on radiographic imaging operation, a radiation irradiation operation apparatus 4 for performing operation relating to irradiation with radiation, a base station 5 for performing communication by a radio communication method such as radio LAN (Local Area Network), and consoles 7a, 7b and 7c for managing the X-ray room 50 and operating the radiographic image detecting apparatus 6 installed in the X-ray room 50 are connected with one another via the network 8 so as to be capable of transmitting and receiving information. The radiation irradiation operation apparatus 4 is connected with a radiation tube 9 for applying radiation to the patient 11 as a subject, through a cable 40. X rays are preferably used as the radiation used for radiographing operation although the radiation is not restricted thereto. When X rays are used as the radiation used for radiographing operation, an X-ray tube for applying X-rays is used as the aforementioned radiation tube 9. An X-ray image detecting apparatus is used as the radiographic image detecting apparatus 6. The consoles 7a, 7b and 7c have the similar structure. In the following description, any one of the consoles 7a, 7b and 7c will be called the console 7.

The number of the consoles linked to the network 8 is not restricted to three. A plurality of further consoles can be linked to the network. The number of the X-ray rooms 50 showing the diagram is not restricted to the illustrated number. A plurality of X-ray rooms 50 can be provided. The X-ray room 50 and console 7 can be associated with each other in one-to-one correspondence. Alternatively, a plurality of X-ray rooms 50 can be associated with one console 7 and a plurality of X-ray rooms 50 can be managed by one console. Alternatively, one X-ray room 50 can be associated with a plurality of consoles 7.

The network 8 can be a communication line specifically designed for this system. However, to prevent the degree of freedom from being reduced in the system configuration, it is preferred to use the existing line such as the Ethernet (registered trademark).

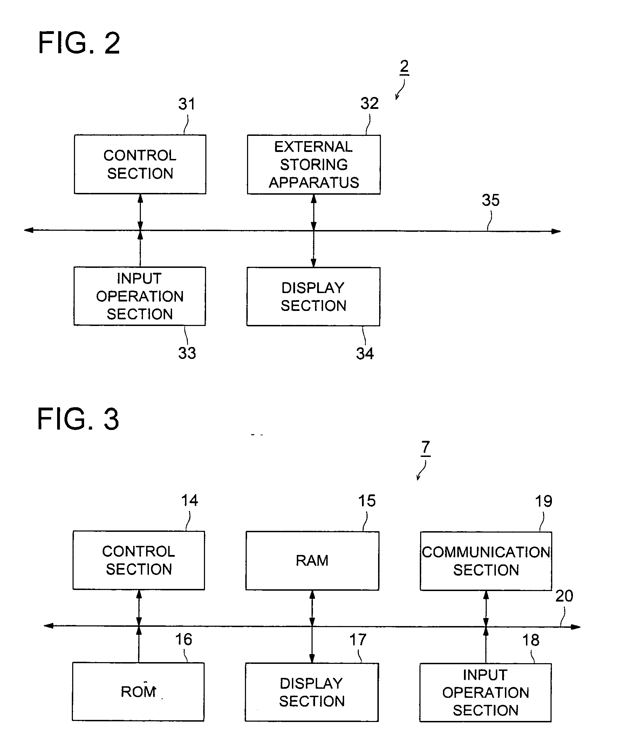

The server 2 is made up of a computer as shown in Fig. 2. The server 2 is provided with a control section 31 for controlling each component of the server 2; an external storage apparatus 32 for storing various forms of information; an input operation section 33 for inputting various forms of information or the instruction of the operator; and a display section 34 for displaying the information having been inputted. The components are connected with one another via the bus 35.

The input operation section 33 is made up, for example, of a keyboard and mouse. The key depression signal resulting from depression of the keyboard or the operation signal resulting from mouse operation as an input signal are outputted to the control section 31.

Especially in the radiation image radiographing system 1 of the present embodiment, the input operation section 33 is designed as a patient information inputting device to input the aforementioned patient information and radiographing information. This system conducts registration of a patient 11 to be imaged. The forms of information inputted through the input operation section 33 are not restricted to the ones shown here.

The control section 31 is made up, for example, of a CPU (Central Processing Unit), and reads various forms of programs stored in the external storage apparatus 32 or the like, whereby various forms of processing are performed, accordingly.

Especially in this embodiment, the control section 31 generates a list (hereinafter referred to as "patient list") which establishes associations of the information inputted through the input operation section 33 and/or the patient information or radiographing information which has been inputted through the unillustrated external apparatus such as HIS (Hospital Information System)/RIS (Radiology Information System) and which has been sent via the network 8. This patient list is stored in the external storage apparatus 32 and others. The patient list generated in the control section 31 of the server 2 is shared by the consoles via the network 8. If new information is inputted through the input operation section 33 and others after generation of a patient list, the patient list is sequentially updated, and the updated patient list is shared by the server 2 and consoles 7.

The patient information represents the information on the patient 11 such as the name, age, sex and birth data of the patient as well as the patient ID number for identifying the patient. The radiographing information refers to the radiographing conditions such as the region to be imaged (the region of the patient's body to be imaged), tube voltage and dose of irradiation (mAs value), and the information required for radiographing operation such as direction and method for radiographing operation. For example, when radiographing operation is performed several times for one patient 11, the intended number of radiographing operations is also inputted as radiographing information.

As described above, the patient information and radiographing information can be the ones inputted through the input operation section 33 of the server 2, or the ones having been inputted from the console 7 and having been sent to the server 2. Further, it is also possible to make such arrangements that the server 2 receives the information inputted through a terminal apparatus such as the other personal computers installed in the doctor's consultation room and linked to the unillustrated HIS (Hospital Information System)/RIS (Radiography Information System) connected to the network 8, for example. It is also possible to arrange such a configuration that an information reading apparatus such as a card reader for reading the patient information or the radiographing information written in the ID card and others in advance is provided, and the patient information or radiographing information is read by such an information reading apparatus, whereby inputting is performed. In this case, the patient information inputting device is made up of the inputting device, information reading apparatus and others.

The radiographic image information having been detected by each radiographic image detecting apparatus 6 and sent to the console 7 is associated with the patient information on the patient having been imaged; the information for identifying the ordinal number of the captured image of the patient to which this radiographic image information belongs; and other information on radiographing. Then, this radiographic image information is sent to the server 2. The radiographic image information sent to the server 2 from the radiographic image detecting apparatus 6 can be associated with the identification information for identifying which of the consoles 7 has been used to operate the radiographic image detecting apparatus 6 used for radiographing operation, for example, at the time of radiographing operation, in addition to the aforementioned information. Such information having been sent, together with the radiographic image information, is associated with the aforementioned patient list by the control section 31, and is stored in the external storage apparatus 32 and others, wherein such information is placed under management.

When the radiographic image information associated with patient information and radiographing information has been sent, the control section 31 conducts a search to see whether or not the radiographic image information of the past associated with the same patient information or the like is included in the radiographic image information stored in the external storage apparatus 32. The radiographic image information of the past in the sense in which it is used here can be all the radiographic image information that is associated with the same patient information and that has been captured so far. For example, when radiographing operation is to be performed several times for one patient, and a series of intended radiographing operations are associated with the patient list, the radiographic image information of the past can be the radiographic image information on the image having been captured already, out of the image information for this patient.

The control section 31 as the image information transmission device ensures that the radiographic image information which is based on the result of detection by the radiographic image detecting apparatus 6 and which is associated with the information on the same patient is sent to the console 7 associated as the console 7 for operating the radiographic image detecting apparatus 6 having been used for the last radiographing operation at the time of radiographing process.

The aforementioned "last radiographing operation" refers to the last of a series of the radiographing operations intended for one patient. To be more specific, when two radiographing operations are planned as a series of radiographing operation for a certain patient, the second radiographing operation is the last one upon completion of the second radiographing operation. Thus, in this case, the radiographic image information obtained in the first radiographing operation is sent to the console 7 associated as the console 7 for operating the radiographic image detecting apparatus 6 used in the second radiographing operation, at the time of radiographing operation. Similarly, when four radiographing operations are planned, the fourth radiographing operation is the last operation at the time of termination of the fourth radiographing operation. The radiographic image information obtained in the first through third radiographing operations is sent to the console 7 associated as the console 7 for operating the radiographic image detecting apparatus 6 used in the fourth operation, at the time of radiographing operation.

Thus, as described above, four radiographing operations are planned for a certain patient, for example. When the radiographic image information associated with the patient information or radiographing information is sent to the control section 31, the control section 31 searches the external storage apparatus 32 based on this patient information or the like. As a result of the search operation, it selects the radiographic image information obtained from the first through third radiographing operations for this patient, and the aforementioned radiographic image information is sent to the console 7 associated as the console 7 used to operate the radiographic image detecting apparatus 6 used for the fourth operation, at the time of radiographing operation.

When, for example, a plurality of operations of radiographing different regions are planned for one patient and a series of planned radiographing operations are associated with the patient list, the radiographic image information to be sent to the console 7 is the information on the radiographing operation already completed out of the planned radiographing operations for this patient. To be more specific, for example, the four planned radiographing operations on the front and side of the abdominal region and the front and side of the pectoral region are associated with the patient list. Further assume that radiographing operations on the front and side of the abdominal region and the front of the pectoral region have been imaged, and the side of the pectoral region is further imaged. In this case, the radiographic image information on the front and side of the abdominal region and the front of the pectoral region which have already been imaged is sent to the console 7 associated as the console 7 for operating the radiographic image detecting apparatus 6 at the time of radiographing operation of the side of the pectoral region.

To observe the chronological change of one region, assume the case wherein a plurality of radiographing operations are planned for one region of one person, for example, such as first, second and third radiographing operations on the abdominal region, or first, second and third radiographing operations on the pectoral region. Also assume that the first and second radiographing operations of the abdominal region and the first and second radiographing operations of the pectoral region have already been performed, and the third radiographing operation of the pectoral region of the patient is carried out, and the radiographic image information thereof, together with the patient information or the like, is sent from the radiographic image detecting apparatus 6. In this case, out of the information on the planned radiographing operations for this patient, only the radiographic image information on the first and second radiographing operations of the pectoral region can be sent to the aforementioned predetermined console 7, wherein this information is related to the same region and to the image having been captured already. Further, the information to be sent can be all the radiographic image information matching the patient information or the like associated with the radiographic image information sent from the control section 31. For example, out of the information matching with the patient information, any number of radiographing operations counted from the youngest one in terms of the radiographing date can be sent.

In this case, both the original image information and image for display can be sent as the radiographic image information sent to the console 7 from the control section 31. However, to ensure smooth transfer of information and quick verification of the image on the console 7, it is preferred to send only the image for display characterized by smaller size of information.

Going back to Fig. 1, the radiation image radiographing apparatus 3 has a radiation tube 9 for applying radiation. This radiation tube 9 is used to apply radiation while having the tube voltage and dose preset on the radiation irradiation operation apparatus 4. An examination table 12 for a patient to lie thereon is installed within the range irradiated with radiation below the radiation tube 9. When a patient 11 is placed on the examination table 12, a radiographic image detecting apparatus 6 for reading the radiation and detecting the radiographic image is arranged at the position on the examination table 12 corresponding to the region of the patient 11 to be imaged. The position wherein the radiographic image detecting apparatus 6 is installed is not restricted to a position between the patient 11 and examination table 12. For example, it is possible to arrange such a configuration that a detecting apparatus installation port (not illustrated) for installing the radiographic image detecting apparatus 6 is arranged below the examination table 12, and the radiographic image detecting apparatus 6 is installed in this detecting apparatus installation port.

The radiation irradiation operation apparatus 4 is made up of a computer including a display section for displaying information, and an input operation section for inputting the instruction from the radiology technician as an operator (both not illustrated). The radiation tube 9 of the radiation image radiographing apparatus 3 is controlled in such a way that the image will be captured at the tube voltage value or radiation dose corresponding to the radiographing conditions having been inputted.

As shown in Fig. 3, the console 7 includes a control section 14, RAM (Random Access Memory) 15, ROM (Read Only Memory) 16, display section 17, input operation section 18, and communication section 19. Each of these components is connected via the bus 20. In this embodiment, as described above, a plurality of consoles 7a, 7b and 7c are connected via the network 8, and each of the consoles 7a, 7b and 7c is provided with a console ID as identification information for identification of the consoles 7a, 7b and 7c, for example.

The display section 17 includes a CRT (Cathode Ray Tube) and LCD (Liquid Crystal Display). It is used to display various forms of screens for the aforementioned patient list, various forms of messages and images and others, in response to the instruction of the display signal sent from the control section 14.

The input operation section 18 includes a keyboard and mouse. The key depression signal operated in response to depression on keyboard and the operation signal resulting from mouse operation are used as input signals, and are outputted to the control section 14. The input operation section 18 can be made up of the so-called touch panel wherein the position information that is inputted by touching the transparent sheet panel that covers the display screen of the aforementioned display section 17, with a finger or special-purpose stylus pen, is used as an input signal and is outputted to the control section 14.

Especially in the radiation image radiographing system 1 of the present embodiment, the input operation section 18 can be used to input the aforementioned patient information and radiographing information as well as the detecting apparatus ID of the radiographic image detecting apparatus 6 and others. The detecting apparatus ID in the sense in which it is used here refers to the identification information of the radiographic image detecting apparatus 6 attached to each radiographic image detecting apparatus 6 for the purpose of identifying the radiographic image detecting apparatus 6.

The console 7 used to input the identification information of radiographic image detecting apparatus 6 is exemplified by the console 7 for managing the X-ray room 50 wherein the radiographic image detecting apparatus 6 is installed. There is no particular restriction to the place where the console 7 for managing the X-ray room 50 is installed. The console 7 is preferably placed closest to the X-ray room 50 or radiation irradiation operation apparatus 4. This arrangement makes it possible to operate the radiographic image detecting apparatus 6 in the X-ray room 50 at the position close to the X-ray room 50 and radiation irradiation operation apparatus 4 at the time of radiographing operation.

Thus, presetting before radiographing operation is made to determine the radiographic image detecting apparatus 6 to be used to image the patient. It is also possible to make such arrangements that, once the aforementioned presetting has been made, this patient cannot be selected on the patient list on another console 7.

The aforementioned display section 17 displays the patient list created according to the patient information or radiographing information inputted from the aforementioned input operation section 18. The input operation section 18 allows selection of the patient to be imaged or the region to be imaged, from this patient list. Further, when there are a plurality of radiographic image detecting apparatuses 6 associated with the console 7, the input operation section 18 allows selection of the particular radiographic image detecting apparatus 6 to be used for radiographing operation. This arrangement permits selection of the particular radiographic image detecting apparatus 6 to be used to image the patient. It is also possible to make such arrangements that, once the aforementioned presetting has been made, this patient cannot be selected on the patient list on another console 7.

The input operation section 18 is used to ensure that the signal for the instruction of transferring the radiographic image information obtained from the result of detection by the radiographic image detecting apparatus 6 is sent to the control section 14 according to a predetermined operation. In the present embodiment, the radiographic image information obtained by radiographing operation is associated with the radiographic image detecting apparatus 6 used at the time of radiographing operation and is sent to the console 7 used to operate the radiographic image detecting apparatus 6. In the control section 14 of the console 7, this information is associated with the patient information and radiographing information of the patient selected from the patient list at the time of the aforementioned setting before the radiographing operation. The radiographic image information having been associated with the patient information and radiographing information is stored in an unillustrated storage apparatus according to requirement, or is sent to the external apparatus such as a server 2.

The input operation section 18 is used in such a way that the signal on the instruction to delete the radiographic image information stored in the radiographic image detecting apparatus 6 is outputted to the control section 14 in response to a predetermined operation.

When the radiology technician has determined as to whether or not the image of the subject is properly detected by the radiographic image detecting apparatus 6, and whether or not there is any need of repeating the radiographing operation, the input operation section 18 is used to input the result of this decision. The result having been inputted is outputted to the control section 14 as an electric signal.

The communication section 19 is used to exchanges various forms of information with the radiographic image detecting apparatus 6 according to the radio communication method such as radio LAN through the base station 5.

Similarly to the case of the control section 31 of the server 2, for example, the control section 14 is made up of a CPU (Central Processing Unit) and others. It reads a predetermined program stored in the ROM (Read Only Memory) 16, expands it in the work area of the RAM (Random Access Memory) 15, and executes various forms of processing according to this program.

In this embodiment, when the detecting apparatus ID as the identification information of the radiographic image detecting apparatus 6 has been inputted from the aforementioned input operation section 18, the detecting apparatus ID having been inputted is sent to the control section 14 as a signal. Upon acquisition of this detecting apparatus ID, the control section 14 establishes association between the detecting apparatus ID and the aforementioned console ID, thereby establishing association between the radiographic image detecting apparatus 6 and console 7 as an association device.

To put it more specifically, for example, when the detecting apparatus ID of the radiographic image detecting apparatus 6 has been inputted from the input operation section 18 of the console 7a, the control section 14 associates this console 7a as a console 7 used to operate the radiographic image detecting apparatus 6 having this detecting apparatus ID at the time of radiographing operation.

The inputting method for identifying the radiographic image detecting apparatus 6 to be associated with the console 7 by the control section 14 is not restricted to the method illustrated here. It is also possible to make such arrangements that, for example, the radiographic image detecting apparatus 6 is assigned with an IC tag or barcode as the identification information for identifying the radiographic image detecting apparatus 6. This is read by a predetermined reading apparatus or the like, whereby the identification information of the radiographic image detecting apparatus 6 is inputted, and this identification information is obtained by the control section 14.

The control section 14 acquires the identification information of the radiographic image detecting apparatus 6 by the input of the detecting apparatus ID through the input operation section 18. Association between the radiographic image detecting apparatus 6 and console 7 is preferably established when the radiographic image detecting apparatus 6 is placed into the X-ray room 50, for example. However, the timing of association is not restricted thereto.

When the radiographic image detecting apparatus 6 having been associated with a certain console 7 has been moved into another X-ray room 50 from the X-ray room 50 where it was initially installed, the detecting apparatus ID of the radiographic image detecting apparatus 6, for example, is inputted from the input operation section 18 of the console 7 for managing the X-ray room 50 at this new place, whereby the association between the radiographic image detecting apparatus 6 and console 7 is re-established by the control section 14 of the console 7 for managing the X-ray room 50 at the new place. When a new relationship of association has been established between the console 7 for managing the X-ray room 50 at the new place and the radiographic image detecting apparatus 6, the previous relationship of association with the console 7 is cancelled.

To put it more specifically, for example, the radiographic image detecting apparatus 6 is placed into a certain X-ray room 50, and the detecting apparatus ID is inputted through the input operation section 18 of the console 7 for managing the X-ray room 50. Then, the control section 14 establishes association with the console 7a as a console 7 to be used for operating the radiographic image detecting apparatus 6 at the time of radiographing operation. After that, the radiographic image detecting apparatus 6 is moved into another X-ray room 50. When the console 7b is managing this X-ray room 50, the detecting apparatus ID of the radiographic image detecting apparatus 6 is inputted from the input operation section 18 of the console 7b, and the control section 14 associates the console 7b as the console 7 to be used to operate this radiographic image detecting apparatus 6 at the time of radiographing operation. When a new relationship of association has been established between this console 7b and radiographic image detecting apparatus 6, and the relationship of association establish previously between the console 7a and radiographic image detecting apparatus 6 is cancelled.

The control section 14 as a patient information association device establishes association between the patient information on the patient having been imaged, and radiographic image information and the radiographing information including the information for identifying the ordinal number of the captured image of the patient to which this radiographic image information belongs; and the radiographic image information based on the result of detection by the radiographic image detecting apparatus 6. The information associated with the radiographic image information by the control section 14 is not restricted to such information. For example, it is also possible to associate the identification information to identify which of the consoles 7 has been used at the time of this radiographing operation to operate with the radiographic image detecting apparatus 6 employed for radiographing operation.

The control section 14 is designed in such a way that the radiographic image information with which the patient information is associated is sent to the server 2 through the network 8.

The control section 14 is designed in such a way that the information required for radiographing operation such as information on the region to be imaged is sent to the radiographic image detecting apparatus 6 by radio through the base station 5.

The radiographic image information acquired by the radiographic image detecting apparatus 6 is sent to the control section 14. Upon receipt of the radiographic image information, the control section 14 as a display image generation device generates a display image as a reduced-size image information whose information amount is smaller than that of the original image information such as the so-called thumbnail image or the like, based on the radiographic image information having been sent. The preferred reduction rate of this reduced size image is such that the length of each side is about 1/2 through 1/100 time (the number of pixels being about 1/4 through 1/10,000 time) of that of the original image, for example. More preferably, the number of pixels is about 1/4 through 1/2,500 time.

Based on the instruction inputted through the input operation section 18, the control section 14 allows various forms of information and images to be displayed on the display section 17, whenever required. It is also possible to make such arrangements that the patient information and radiographing information related to the next radiographing operation are displayed on the display section 17, if an instruction has been inputted through the input operation section 18 that the re-radiographing operation is not to be performed.

Further, based on the signal on the deletion instruction having been inputted from the aforementioned input operation section 18, the control section 14 operates in such a way that the signal for instructing deletion of the result of detection stored in the radiographic image detecting apparatus 6 is sent to the radiographic image detecting apparatus 6. The result of detection is then deleted. In this case, it is also possible to make such arrangements that the previous relationship of association with the console 7 is reset.

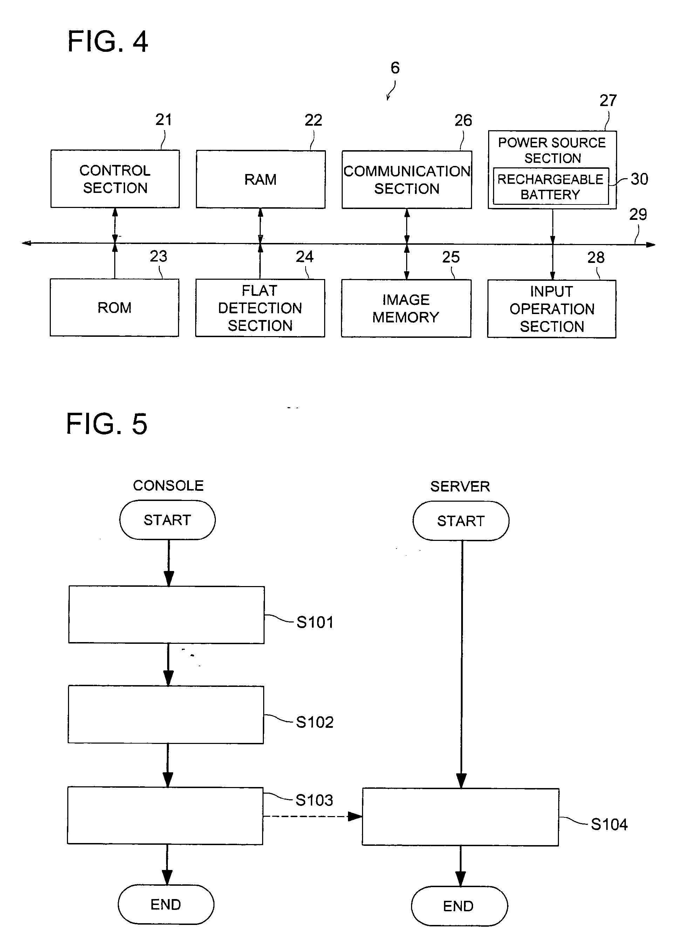

The radiographic image detecting apparatus 6 is a portable cassette type FPD, for example. As shown in Fig. 4, the radiographic image detecting apparatus 6 includes a control section 21, RAM 22, ROM 23, flat detection section 24 as a radiation detection section, image memory 25 as a storage device, communication section 26, power source section 27, and input operation section 28. Each section is connected through a bus 29.

The radiographic image detecting apparatus 6 is assigned with a detecting apparatus ID as identification information for identification of the radiographic image detecting apparatus 6. As described above, the identification information for identification of the radiographic image detecting apparatus 6 is not restricted to the detecting apparatus ID alone. For example, an IC tag and barcode can be used.

The flat detection section 24 has a glass substrate, for example. A plurality of pixels are arranged in a matrix at a predetermined position on the substrate, wherein these pixels serve to detect the radiation having been applied from the radiation tube 9 and at least having passed through the subject, according to the intensity thereof, and to convert the detected radiation into an electric signal, and to store this signal.

The flat detection section 24 is exemplified by an indirect type containing a radiation-photo conversion layer for converting radiation to fluorescent light (light), and a photoelectric conversion layer for detecting the fluorescence obtained from conversion by the radiation-photo conversion layer and converting the fluorescence into en electric signal; and a direct type containing a radiation electric signal conversion layer having a radiation receiving section for converting radiation directly into electric charges, instead of the radiation-photo conversion layer and photoelectric conversion layer, although they are not illustrated. In this case, the indirect type does not require a high voltage power source which is used in the direct type, and is more preferably used.

The image memory 25 is made up of a nonvolatile memory such as a flash memory, and an RAM, for example, and is capable of storing the radiographic image information equivalent to a plurality of radiographing operations, this radiographic image information being obtained by reading the electric signal stored in the flat detection section 24.

The communication section 26 exchanges various forms of information with the console 7 through the base station 5 according to the radio communication method such as radio LAN. For example, the communication section 26 receives the patient information and radiographing information sent from the communication section 19 of the console 7 as well as various forms of information such as console ID of the console 7 associated in relation to the radiographic image detecting apparatus 6. Further, the communication section 26 sends the detecting apparatus ID of this radiographic image detecting apparatus 6 to the console 7. It also sends the radiographic image information stored in the image memory 25 to the console 7 associated in relation to the radiographic image detecting apparatus 6. Further, the communication section 26 receives the radiographing operation suitability signal for determining the suitability of the radiographic image having been sent through the communication section 19 of the console 7, and the deletion instruction signal for instructing the deletion of the result of detection stored in the radiographic image detecting apparatus 6.

The power source section 27 has a rechargeable battery 30 for supplying power to each component of the radiographic image detecting apparatus 6, and is designed as rechargeable through the recharging terminal (not illustrated) provided at a predetermined position of the radiographic image detecting apparatus 6.

The control section 21 is made up of a CPU, for example. It reads out a predetermined program stored in the ROM 23, expands it in the work area of the RAM 22, and executes various forms of processing according to the program.

To put it more specifically, for example, the control section 21 controls the switching section constituting each pixel of the flat detection section 24 such as the TFT (Thin Film Transistor), switches the readings of the electric signal stored in each of these pixels, and reads the electric signal stored in the flat detection section 24, whereby the result of detection is obtained from the flat detection section 24.

Further, the control section 21 controls the communication section 26 in such a way that the detecting apparatus ID of the radiographic image detecting apparatus 6 is sent to the console 7. The control section 21 also controls the communication section 26 in such a way that the radiographic image information based on the result of detection having been detected by the radiographic image detecting apparatus 6 as the image information transmission device is sent to the console 7.

In this embodiment, the receiver of the radiographic image information sent from the control section 21 through the communication section 26 is the console 7 associated as the console 7 for operating this radiographic image detecting apparatus 6 at the time of radiographing operation. The radiographic image information sent to the console 7 is provided with the patient information and radiographing information associated with the patient list, the identification information such as the detecting apparatus ID of the radiographic image detecting apparatus 6 used for radiographing operation, and the accompanying information such as the console ID of the console 7 associated as the console 7 for operating the radiographic image detecting apparatus 6 at the time of radiographing operation. It is also possible to make such arrangements that the information of the timing of transferring this radiographic image information from radiographic image detecting apparatus 6 can be inputted from the input operation section 28. This radiographic image information is preferably transferred from radiographic image detecting apparatus 6 at the earliest possible timing.

The following describes the operation of the radiation image radiographing system 1:

Referring to Fig. 5, the following describes the operation of the console 7 and server 2 when a new radiographic image detecting apparatus 6 is installed in an X-ray room 50. The identification information such as the detecting apparatus ID of the radiographic image detecting apparatus 6 is inputted from the input operation section 18 of the console 7 for managing the X-ray room 50 by a radiology technician (Step S101). When the identification information of the detecting apparatus ID has been inputted, association is established between the console 7 and radiographic image detecting apparatus 6 by the control section 14 (Step S102). When the association has been established, the console ID of the console 7 associated with the detecting apparatus ID of the radiographic image detecting apparatus 6 is sent to the server 2 via the network 8 (Step S103), and is stored in the external storage apparatus 32 of the server 2 or the like (Step S104), whereby the operation terminates.

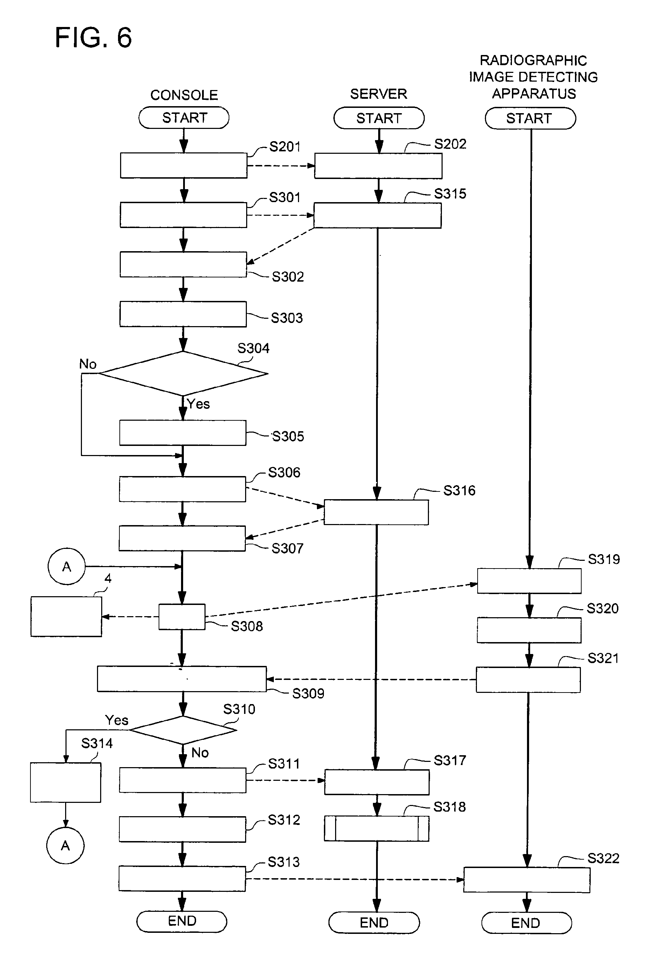

Referring to Fig. 6, the following describes the operation of the first embodiment of the operation at the time of radiographing operation in the radiation image radiographing system of the present invention.

When any of the patient information, radiographing information and the like has been inputted from the console 7 (Step S201), the inputted information is sent to the server 2 via the network 8. When the patient information or radiographing information has been sent, the patient list associated with such information is generated by the control section 31 of the server 2 (Step S202). In addition to being inputted from the console 7 in Step S201, the patient information, radiographing information or the like can be sent from the input operation section 33 of the server 2, various forms of information reading apparatus such as an ID card reader, or the terminal apparatus linked to the HIS/RIS installed in the consultation room which is linked with the network 8.

To start radiographic imaging operation, the radiology technician goes to the console 7 associated with the X-ray room 50 wherein radiographing operation is to be performed, and refers to the patient list stored in the server 2 via the network 8 (Step S301) to take action so that the patient list is sent to the console 7a from the server 2 (Step S315), and the patient list is displayed on the display section 17 of the console 7a (Step S302). The radiology technician selects the patient 11 as an object of radiographing operation and the region of the patient as required (selection of the order of radiographing operations) (Step S303) therefrom. If a plurality of radiographic image detecting apparatuses 6 are installed in the X-ray room 50 (Yes in Step S304), the radiology technician uses the input operation section 18 to select and input the radiographic image detecting apparatus 6 to be used for the radiographing operation, out of the radiographic image detecting apparatuses 6 installed in the X-ray room 50 (Step S305).

The radiographing order ID corresponding to the order of radiographing operation selected by the Steps S303 through S305 is sent from the console 7a to the server 2 (Step S306). In response to the processing in Step S306, the patient information and radiographing information associated with the radiographing order ID having been sent in Step S306 is sent to the console 7a and radiation irradiation operation apparatus 4 from the server 2 (Step S316). The patient information and radiographing information is displayed on the display section 17 of the console 7 appropriately (Step S307), and the radiology technician performs radiographic imaging operation by verifying such information (Step S308).

Based on the tube voltage value and radiation dose contained in the radiographing information having been received, the radiation irradiation operation apparatus 4 controls radiation tube 9 of the radiation image radiographing apparatus 3, and the radiation image radiographing apparatus 3 irradiates the patient 11 with radiation under these conditions.

In this case, the radiographic image detecting apparatus 6 is located on the examination table 12 and is inserted below the patient 11. It detects the dose of radiation passing through the patient 11 (Step S319), and converts the radiation having been detected, into electric signal, thereby acquiring a predetermined result of detection and storing this result of detection in the image memory 25 (Step S320).

The control section 21 provides control in such a way that the result of detection detected by the radiographic image detecting apparatus 6 in the state associated with the detecting apparatus ID of radiographic image detecting apparatus 6 used for radiographing operation is sent to the console 7a associated with the radiographic image detecting apparatus 6, as a console 7 to operate the radiographic image detecting apparatus 6 at the time of radiographing operation (Step S321).

Based on the result of detection having been sent, the control section 14 of the console 7a as a display image generation device generates the display image such as so-called the thumbnail image whose size of information is smaller than that of the original image information. The display image having been generated is displayed on the display section 17 (Step S309). The radiology technician verifies the display image and checks whether or not the subject image is properly detected by the radiographic image detecting apparatus 6, and whether or not there is any need for repeating the radiographing operation (Step S310).

When the subject image is properly detected and there is no need for repeating the radiographing operation (No in Step S310), the control section 14 of the console 7a establishes association between original image information, patient information and other information through the communication section 19, and sends the result to the server 2 (Step S311). After that, the original image information is subjected to image processing by the server 2 (Step S317), and is then outputted from the printer or monitor appropriatly. It is used as a radiographic image for analysis by a doctor (Step S318). The display image is deleted by the control section 14 (Step S312).

The control section 14 of the console 7a sends to the radiographic image detecting apparatus 6 the signal representing the adequacy of the radiographic imaging operation and the deletion instruction signal for instructing deletion of the result of detection from the radiographic image detecting apparatus 6 (Step S313). In response to these signals, the control section 21 of the radiographic image detecting apparatus 6 deletes the result of detection from the image memory 25 (Step S322), and terminates the radiographing operation using the radiographic image detecting apparatus 6. In this case, the display section 17 of the console 7a displays the patient information, radiographing information and other information for the next radiographing operation, as appropriate.

By contrast, if a signal is inputted from the input operation section 18 to indicate that the subject image is not properly detected and the radiographing operation must be repeated (Yes in Step S310), the original image information and display image are deleted (Step S314), and an instruction is sent to the radiation irradiation operation apparatus 4 to repeat radiographing operation (Step S308). This procedure allows the radiographing operation to be repeated, and the result of detection detected by the radiographic image detecting apparatus 6 is again sent to the console 7 (Step S321). Based on the result of detection, the control section 14 of the console 7a generates the display image whose size of information is smaller than that of the original image information. The display image having been generated is displayed on the display section 17. If the subject image is detected properly and there is no need of repeating the radiographing operation, the control section 14 of the console 7a establishes association between the original image information, patient information and other information through the communication section 19. The resulting information is then sent to the server 2. Similarly to the aforementioned case, the control section 14 of the console 7a deletes the display image and sends to the radiographic image detecting apparatus 6 the signal representing the adequacy of the radiographic imaging operation and the deletion instruction signal for instructing deletion of the result of detection from the radiographic image detecting apparatus 6, whereby a series of radiographing operations terminate.

When radiographic image detecting apparatus 6 is moved into another X-ray room 50 after that, the identification information of the detecting apparatus ID or the like of the radiographic image detecting apparatus 6 is inputted from the input operation section 18 of the console 7b managing this X-ray room 50 at the time of movement into the X-ray room 50, for example. Then the same processing as that shown in Fig. 5 is executed in the console 7 and server 2. To be more specific, when the identification information of the radiographic image detecting apparatus 6 has been inputted, association between the console 7b and radiographic image detecting apparatus 6 is established by the control section 14 of the console 7b, and the association previously established between the console 7a and radiographic image detecting apparatus 6 is cancelled. When the console 7b is associated with the radiographic image detecting apparatus 6, the console ID of the console 7b and the detecting apparatus ID of the radiographic image detecting apparatus 6 in the state associated with each other are sent to the server 2 through the network 8, and this new relationship of association is stored in the external storage apparatus 32 of the server 2.

If the next radiographing operation is planned in this new X-ray room 50, the radiographic image detecting apparatus 6 is operated through the console 7b at the time of radiographing operation, and the result of detection detected by the radiographic image detecting apparatus 6 is sent to the console 7b. Further, a display image is generated by the control section 14 of the console 7b and is displayed on the display section 17 of the console 7b.

It is also possible to make such arrangements that the radiographing operation is performed several times by moving between the X-ray rooms 50. In this case, before starting each radiographing operation, association is established between the radiographic image detecting apparatus 6 used for radiographing operation and the console 7 for operating the radiographic image detecting apparatus 6, and the result of detection obtained by the radiographic image detecting apparatus 6 is sent to the console 7 associated at the time of each radiographing operation.

According to the aforementioned procedure in the radiation image radiographing system 1 of the present embodiment, the result of detection obtained by the radiographic image detecting apparatus 6 is sent to the console 7 in front of the X-ray room 50 associated in advance, at the time of radiographing operation. A display image is generated by the console 7, and is displayed on the display section of the console 7. Thus, independently of the consoles 7 used to input the patient information or radiographing information and to establish association between the radiographic image detecting apparatus 6 and console 7, the image having been captured can be verified on the console 7 located close to the X-ray room 50 used for this radiographing operation. Without having to move far away from the site of radiographing operation, the radiology technician is allowed to check for proper detection of the subject image immediately and correctly, and to determine the suitability of the radiographic image.

In the present embodiment, the result of detection detected by the radiographic image detecting apparatus 6 is sent to the corresponding console 7, and the display image was generated by the control section 14 of the console 7. However, the display image generation device for generating the display image is not necessarily restricted to the control section 14 of the console 7.

For example, when the radiographic image detecting apparatus 6 is a cassette type FPD as in this embodiment, it is possible to make such arrangements that the display image is generated by the control section 21 of the radiographic image detecting apparatus 6. In this case, the original image information and display image generated by the control section 21 of the radiographic image detecting apparatus 6 can be sent to the console 7 or to the server 2 by a radio-or otherwise-based communication device. It is also possible to make such arrangements that only the display image is sent to the console 7, and the original image information is sent to the server 2. It is also possible to make such arrangements that the original image information is stored in the image memory 25 of the radiographic image detecting apparatus 6, and only the display image is sent to the console 7. In this case, when a signal has been sent to the radiographic image detecting apparatus 6 from the console 7 to notify that the radiographic image is properly captured, the original image information can be sent to the console 7 or server 2. Further, the result of detection by the radiographic image detecting apparatus 6 is directly sent to the server 2, and a display image can be generated in addition to the original image information in the server 2.

In the present embodiment, the control section 21 of the radiographic image detecting apparatus 6 as the image information transmission device sends the radiographic image information to a predetermined console 7 through the communication section 26. However, the image information transmission device is not restricted thereto. For example, as described above, when the result of detection by the radiographic image detecting apparatus 6 is directly sent to the server 2, and a display image is generated in addition to the original image information in the server 2, the control section 31 of the server 2 as the image information transmission device sends the original image information and display image, or only the display image to the console 7.

In this case, the result of detection of the radiographic image detecting apparatus 6 is sent to the server 2, for example, in the state associated with the detecting apparatus ID of the radiographic image detecting apparatus 6 used in this radiographing operation. The control section 31 of the server 2 checks matching of the detecting apparatus ID having been sent, with the detecting apparatus ID stored in the external storage apparatus 32, and sends the radiographic image information to the console 7 containing the console ID associated with the matching detecting apparatus ID. It is also possible to arrange such a configuration that the result of detection sent to the server 2 from the radiographic image detecting apparatus 6 is associated with the console ID of the console 7 used to operate the radiographic image detecting apparatus 6 at the time of radiographing operation, in addition to the detecting apparatus ID of radiographic image detecting apparatus 6 used in this radiographing operation. In this case, the radiographic image information is sent to the console 7 associated with this console ID.

In the present embodiment, patient list is generated by the control section 31 of the server 2. The generating device of the patient list is not restricted thereto. For example, it is possible to arrange such a configuration that the patient list is generated by the control section 14 of the console 7, and is sent from the console 7 to the server 2. Then, it is stored in the external storage apparatus 32 of the server 2. The patient list is sent to each of the consoles 7 from the server 2, and is shared among them.

In the present embodiment, after the radiographic image detecting apparatus 6 has been associated with a certain console 7, it is moved between the X-ray rooms 50 and is associated with a new console 7. In this case, the previous relationship of association between the console 7 and radiographic image detecting apparatus 6 is cancelled. However, it is possible to arrange such a configuration that, when it is moved between the X-ray rooms 50 and is associated with a new console 7, the previous relationship of association is kept without being cancelled, and a plurality of overlapped relationships of association is present. In this case, for example, it is also possible to make such arrangements that the radiographic image obtained by the radiographic image detecting apparatus 6 is associated with the console ID of the console 7 used to operate the radiographic image detecting apparatus 6 at the time of this radiographing operation, whereby this radiographic image is sent to only the console 7 containing this console ID. Thus, this image can be sent to only the console 7 used to operate the radiographic image detecting apparatus 6 at the time of this radiographing operation. After radiographing operation, the image can be verified immediately close to the X-ray room 50 for the convenience of the operator.

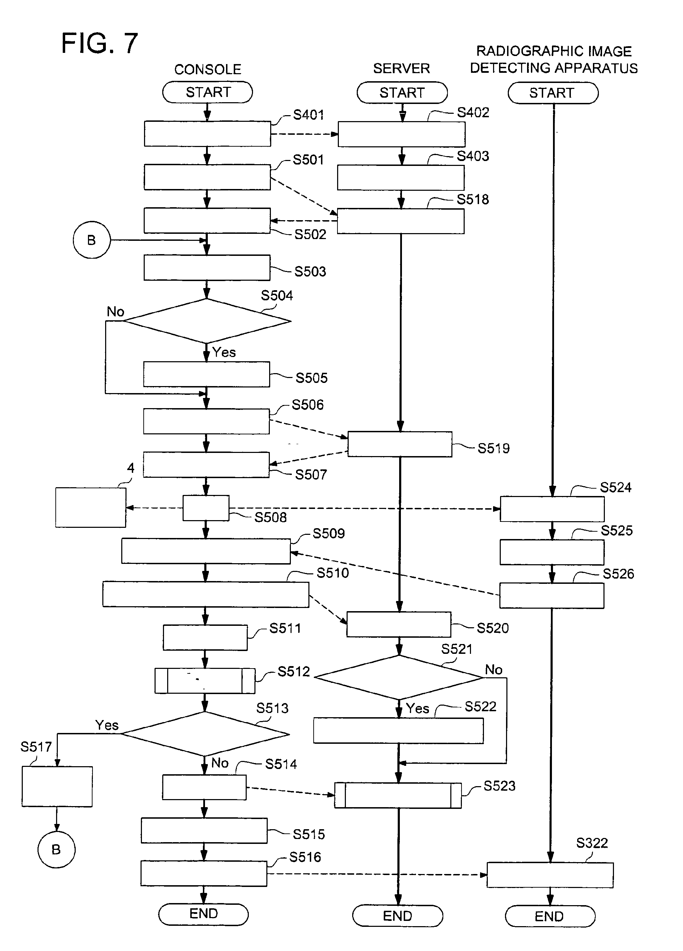

Referring to Fig. 7, the following describes the second embodiment of the operation at the time of radiographing operation using a radiation image radiographing system of the present invention. In the second embodiment, the difference from the first embodiment is that the radiographing image of the same patient is searched in the server 2 at every radiographing operation, display image data in all the radiographing operations for the same patient is sent to any one of the consoles 7a, 7b and 7c that has issued the radiographing operation instruction, and all the images are displayed on the consoles 7a, 7b and 7c that has issued the radiographing operation instruction.

When the patient information and radiographing information have been inputted from any one of the consoles 7 (Step S401), the information having been inputted is sent to the server 2 through the network 8. In this case, the planned number of radiographing operations is also inputted as the radiographing information. For example, when four radiographic imaging operations are to be performed for each patient, radiographing operations are registered as four operations. The number of radiographing operations and other information can be modified as appropriate by inputting addition or correction through the input operation section 18 or server 2 in a later step.

When the patient information and radiographing information has been sent from the server 2, a patient list associated with various forms of information is generated by the control section 31 of the server 2 (Step S402), and is stored in the external storage apparatus 32 of the server 2 (Step S403). In addition to being inputted through the console 7 as shown in Step S401, the patient information and radiographing information can be inputted from the operation section 33 of the server 2, various types of information reading apparatus such as an ID card reader, or the terminal apparatus linked with the HIS/RIS which is installed in the consultation room and is linked with the network 8.

To start radiographic imaging operation, the radiology technician goes to the console 7a which is installed in the X-ray room 50 wherein radiographing operation is to be performed, and which is already associated by the processing of association shown in Fig. 5, and refers to the patient list stored in the server 2 via the network 8 (Step S501). Then, the radiology technician takes action so that the patient list is sent to the console 7a from the server 2 (Step S518) and the patient list is displayed on the display section 17 of the console 7a (Step S502). The radiology technician selects the patient 11 as an object of radiographing operation and the region of the patient to be imaged as required (selection of the order of radiographing operations) (Step S503) therefrom. If a plurality of radiographic image detecting apparatuses 6 are installed in the X-ray room 50 (Yes in Step S504), the radiology technician uses the input operation section 18 to select and input the radiographic image detecting apparatus 6 to be used for the radiographing operation, out of the radiographic image detecting apparatuses 6 installed in the X-ray room 50 (Step S505).

The radiographing order ID corresponding to the order of radiographing operation selected in Steps S503 through S505 is sent from the console 7a to the server 2 (Step S506). In response to the processing in Step S506, the patient information and radiographing information associated with the radiographing order ID sent in Step S506 are sent to the console 7a and radiation irradiation operation apparatus 4 from the server 2 (Step S519). The patient information and radiographing information are displayed on the display section 17 of the console 7a as appropriate (Step S507), and the radiology technician performs radiographic imaging operation by checking such information (Step S508).

Based on the tube voltage value and radiation dose contained in the radiographing information having been received, the radiation irradiation operation apparatus 4 controls the radiation tube 9 of the radiation image radiographing apparatus 3, and the radiation image radiographing apparatus 3 irradiates the patient 11 with radiation under this condition.

In this case, the radiographic image detecting apparatus 6 is located on the examination table 12 and is inserted below the patient 11. It detects the dose of radiation passing through the patient 11 (Step S524), and converts the radiation having been detected, into electric signal, thereby acquiring radiographic image information based on a predetermined result of detection. This radiographic image information is stored in the image memory 25 (Step S525).

The control section 21 provides control in such a way that the radiographic image information obtained by the radiographic image detecting apparatus 6 in the state associated with the detecting apparatus ID of radiographic image detecting apparatus 6 used for radiographing operation is sent to the console 7a associated with the radiographic image detecting apparatus 6, as a console 7 to operate the radiographic image detecting apparatus 6 at the time of radiographing operation (Step S526).

Based on the result of detection having been sent, the control section 14 of the console 7a as a display image generation device generates the display image such as so-called the thumbnail image whose size of information is smaller than that of the original image information (Step S509). At the same time, it establishes the association between the patient information and the radiographic image information by the radiographic image detecting apparatus 6, and sends the radiographic image information including the display image in the state associated with the patient information, to the server 2 through the network 8 (Step S510).

The server 2 establishes association between the radiographic image information having been sent, and the patient information, and stores it in the external storage apparatus 32 (Step S520).

The control section 14 of the console 7a allows the generated display image to be displayed on the display section 17 (Step S511). The radiology technician checks the display image, thereby determining whether or not the subject image is properly detected by the radiographic image detecting apparatus 6, and whether or not there is any need for repeating the radiographing operation (Step S512).

To perform radiographing operation of still another region for the same patient in the same X-ray room 50 (Yes in Step S513), the process goes back to the Step S502, and the radiology technician allows the display section 17 of the console 7a to display the patient list again. The radiology technician selects the region for the second radiographing operation therefrom (Step S503).

When the_radiographing operation has been performed in the manner similar to that of the first radiographing operation, the radiographic image information is stored in the image memory 25. Associated with the detecting apparatus ID of the radiographic image detecting apparatus 6 used for radiographing operation, this information is sent by the control section 21 to the console 7a associated with the radiographic image detecting apparatus 6 as the console 7 which is used to operate the radiographic image detecting apparatus 6 at the time of radiographing operation (Steps S524 through S526).

The control section 14 of the console 7a establishes association between the patient information and radiographic image information by the radiographic image detecting apparatus 6. Based on the result of detection having been sent, the control section 14 of the console 7a as a display image generation device generates the display image such as so-called the thumbnail image whose size of information is smaller than that of the original image information (Step S509). It sends the radiographic image information associated with the patient information to the server 2 (Step S510). The server 2 establishes association between the radiographic image information having been sent, and the patient information, and stores it in the external storage apparatus 32 (Step S520). A search is made to check whether or not radiographic image information matching the patient information associated with the radiographic image information having been sent is stored in the external storage apparatus 32 (Step S521). In the present embodiment, as a result of the search, out of the radiographic image information obtained by the first radiographing operation of the patient, the display image is regarded as matching the patient information (Yes in Step S521), and is sent to the console 7a (Step S522).

The timing for the control section 31 of the server 2 to search whether or not any information matching the patient information of the patient radiographed at the operation under the control of the console 7a is included in the radiographic image information stored in the external storage apparatus 32 is not restricted to the time when the radiographic image information is sent from the console 7a. For example, it is also possible to make such arrangements that, when the same patient 11 to be imaged and the region to be imaged have been selected from the patient list by the radiology technician, this selection signal is sent to the server 2, and a search is performed, and then the display image selected by the search is sent to the console 7a.

The control section 14 of the console 7a ensures that the display image generated in response to radiographic image information of the second radiographing operation is displayed on the display section 17 (Step S511). In this case, the display images obtained from the first radiographing operation are displayed side by side on the display section 17, and the radiology technician checks two display images, thereby determining whether or not the subject image is properly detected by the radiographic image detecting apparatus 6, and whether or not there is any need for repeating the radiographing operation (Step S512).

When the radiographic image detecting apparatus 6 is moved into a different X-ray room 50, and a different region of the same patient is subjected to radiographing operation (Yes in Step S513), the detecting apparatus ID of the radiographic image detecting apparatus 6 is registered into the console 7b that manages the X-ray room 50 when the radiographic image detecting apparatus 6 is installed in the X-ray room 50, and the relationship of association is established (Step S517). Then, the process goes back to the Step S502, and the patient list is displayed on display section 17 of this console 7b. Then, the radiology technician selects from the list the region of this patient 11 for the third radiographing operation (Step S503).

When radiographing operation has been performed in the same manner as the first and second operations, radiographic image information is stored in the image memory 25. The control section 21 provides control in such a way that this image information associated with the detecting apparatus ID of the radiographic image detecting apparatus 6 used for the radiographing is sent to the console 7b associated with the radiographic image detecting apparatus 6 as a console 7b for operating the radiographic image detecting apparatus 6 at the time of radiographing operation (Steps S524 through S526).

The control section 14 of the console 7b establishes association between patient information and radiographic image information by the radiographic image detecting apparatus 6. Based on the result of detection having been sent, the control section 14 as the display image generation device generates the display image such as so-called thumbnail image whose size of information is smaller than that of the original image information (Step S509). The radiographic image information associated with the patient information is_sent to the server 2 (Step S510). The server 2 establishes association between the radiographic image information having been sent and the patient information, and stores it in the external storage apparatus 32 (Step S520). Further, the control section 31 of the server 2 performs a search to find out whether or not the radiographic image information stored in the external storage apparatus 32 contains any information that matches the patient information associated with the radiographic image information sent from the console 7b (Step S521). If there is any matching information (Yes in Step S521), the display image thereof is sent to the console 7b (Step S522). In the present embodiment, as a result of search, the radiographic image information obtained by the first and second radiographing operations of the patient is regarded as matching the patient information, and the display image thereof is sent to the console 7b.

The control section 14 of the console 7b ensures that the display image generated from the radiographic image information obtained from the third radiographing operation is displayed on the display section 17 (Step S511). The display section 17 displays the display images of the first and second radiographing operations sent from the server 2, side by side with the display image of the third radiographing operation. The radiology technician checks three display images, thereby determining whether or not the subject image is properly detected by the radiographic image detecting apparatus 6, and whether or not there is any need of repeating the radiographing operation (Step S512).

Further, when only the patient and radiology technician are moved into a different X-ray room 50, and a different region of the patient is to be imaged by a radiographic image detecting apparatus 6 which is different from that used in the first through third radiographing operations and which is associated with the console 7c for managing this new X-ray room 50, a patient list is displayed on the display section 17 of the console 7c (Step S502), and the radiology technician selects from this list the imaged region of this patient 11 to be imaged in the fourth radiographing operation (Step S503).

When radiographing operation has been performed in the same manner as the first through third operations, radiographic image information is stored in the image memory 25. The control section 21 provides control in such a way that this image information in the state associated with the detecting apparatus ID of the radiographic image detecting apparatus 6 used for the radiographing is sent to the console 7c associated with the radiographic image detecting apparatus 6 as a console 7 for operating the radiographic image detecting apparatus 6 at the time of radiographing operation (Steps S524 through S526).

The control section 14 of the console 7c establishes association between patient information and radiographic image information by the radiographic image detecting apparatus 6. Based on the result of detection having been sent, the control section 14 as the display image generation device generates the display image such as so-called thumbnail image whose size of information is smaller than that of the original image information (Step S509). The radiographic image information associated with the patient information is sent to the server 2 (Step S510). The server 2 establishes association between the radiographic image information having been sent and the patient information, and stores it in the external storage apparatus 32 (Step S520). Further, the control section 31 of the server 2 performs a search to find out whether or not the radiographic image information stored in the external storage apparatus 32 contains any information that matches the patient information associated with the radiographic image information sent from the console 7c. If there is any matching information (Yes in Step S521), the display image thereof is sent to the console 7c (Step S522). In the present embodiment, as a result of search, the radiographic image information obtained by the first through third radiographing operations of the patient is regarded as matching the patient information, and the display image thereof is sent to the console 7c.

The control section 14 of the console 7c ensures that the display image generated from the radiographic image information obtained from the fourth radiographing operation is displayed on the display section 17 (Step S511). The display section 17 displays the display images of the first through third radiographing operations sent from the server 2, side by side with the display image of the fourth radiographing operation. The radiology technician checks four display images, thereby determining whether or not the subject image is properly detected by the radiographic image detecting apparatus 6, and whether or not there is any need of repeating the radiographing operation (Step S512).

As described above, the radiology technician checks whether or not the subject image is properly detected, and whether or not there is any need of repeating the radiographing operation. If it has been found out, as a result of this check, that the subject image is properly detected, and there is no need of repeating the radiographing operation, the radiology technician uses the input operation section 18 to input the information that the radiographing operation has been performed properly. After this information has been inputted, control section 14 of the console 7 sends a signal to the server 2 to notify that radiographing operation has been made properly (Step S514). Having been subjected to image processing by the server 2, the original image information is outputted from such an output device as a printer or monitor as appropriate and is supplied to the doctor for diagnosis as a radiographic image (Step S523). In the meantime, the display image is deleted by the control section 14 (Step S515).

The control section 14 sends to the radiographic image detecting apparatus 6 a signal notifying that the conditions of the radiographic image are proper, and a deletion instruction signal which instructs deletion of the radiographic image information which is stored in the radiographic image detecting apparatus 6 (Step S516). In response to this signal, the control section 21 of the radiographic image detecting apparatus 6 deletes the radiographic image information from the image memory 25 (Step S322), whereby the radiographing operation by the radiographic image detecting apparatus 6 is terminated. In this case, the process goes back to the Step S501, and the patient information, radiographing information and other information on the next radiographing operation are displayed on the display_section 17 of the console 7, as appropriate.

By contrast, if a signal has been inputted from the input operation section 18 notifying that the subject image is not properly detected and the radiographing operation has to be repeated, the original image information and display image are deleted, and a signal is sent to the radiation irradiation operation apparatus 4 to repeat radiographing operation. Then, radiographing operation is repeated, and the radiographic image information obtained from the radiographic image detecting apparatus 6 is sent to the console 7 used to operate the radiographic image detecting apparatus 6 at the time of radiographing operation, similarly as described above. This procedure is the same as that of Step S310 and Step S314 shown in Fig. 6. In Fig. 7, detailed description is omitted in Step S512.

The control section 14 of the console 7 establishes association between the radiographic image information and patient information and sends the result to the server 2. Based on the radiographic image information, the control section 14 generates the display image whose size of information is smaller than that of the original image information. This display image is then displayed on the display section 17.

If a signal has been inputted from the input operation section 18 notifying that the subject image is properly detected and the radiographing operation need not be repeated, the control section 14 of the console 7 deletes the display image similarly to the above, and sends to the radiographic image detecting apparatus 6 a signal notifying that the conditions of the radiographic image are proper, and a deletion instruction signal and which instructs deletion of the radiographic image information which is stored in the radiographic image detecting apparatus 6, whereby a series of radiographing operations terminate.

Thus, according to the radiation image radiographing system 1 of the present embodiment, all the radiographic image information captured from this patient is sent to the console 7 associated as the one operating the radiographic image detecting apparatus 6 at the time of the final radiographing operation of the patient, and the display image is displayed on the display section of the console 7. Thus, independently of the console 7 used to input the patient information and radiographing information or to establishes association between the radiographic image detecting apparatus 6 and console 7, the suitability of all radiographing operations for captured images can be verified using the console 7 located close to the X-ray room 50 wherein the radiology technician is currently performing the radiographing operation. Without having to move far away from the site of radiographing operation, the radiology technician is allowed to check for proper detection of the subject image immediately and certainly, and to determine the suitability of the radiographic image.

In the present embodiment, the timing for the control section 31 of the server 2 to search the external storage apparatus 33 for the radiographic image information associated with the same patient information is the time when the radiographic image information associated with the patient information is sent from the console 7, as described above. However, this timing is not restricted thereto. For example, it is also possible to make such arrangements that, when a patient to be imaged is selected from the patient list in the step previous to radiographing operation, this information is sent to the server 2, and a search is started by the control section 31.

In the present embodiment, a plurality of display images sent to the console 7 are displayed side by side on the display section 17 of the console 7, witht the method of display not being restricted thereto. For example, it is possible to make such arrangements that a display image is switched over to another as appropriate and is displayed on the display section 17.

In the present embodiment, after the radiographic image information based on the result of detection by the radiographic image detecting apparatus 6 is sent to the console 7 corresponding to the radiographic image detecting apparatus 6, a display image is generated by the control section 14 of the console 7. However, the display image generation device for generating the display image is not restricted to the control section 14 of the console 7.

For example, as in the present embodiment, when the radiographic image detecting apparatus 6 is a cassette type FPD, the display image can be generated by the control section 21 of the radiographic image detecting apparatus 6. In this case, the original image information and display image generated by the control section 21 of the radiographic image detecting apparatus 6 can both be sent to the console 7 or server 2 by a radio communication device or the like. Further, it is possible to make such arrangements that only the display image is sent to the console 7, and the original image information is sent to the server 2. It is also possible to make such arrangements that the original image information is stored in the image memory 25 of the radiographic image detecting apparatus 6, and only the display image is sent to the console 7. In this case, when a signal is sent from the console 7 to the radiographic image detecting apparatus 6 notifying that radiographic imaging conditions are proper, the original image information can be sent to the console 7 or server 2. It is also possible to arrange such a configuration that the radiographic image information obtained by the radiographic image detecting apparatus 6 is directly sent to the server 2, where the display image in addition to the original image information is generated.

In the present embodiment, after all the results of detection detected by the radiographic image detecting apparatus 6 have been sent to the console 7, they are sent to the server 2. However, they can be sent directly to the server 2 from the radiographic image detecting apparatus 6. In the radiographic image detecting apparatus 6, it is possible to arrange such a configuration that, when the display image is generated based on the results of detection, only the display image is sent to the console 7, and the original image information is stored in the image memory 25 of the radiographic image detecting apparatus 6. It is also possible to send only the display image to the console 7, and to send the original image information to the server 2. When the original image information is stored in the image memory 25 of the radiographic image detecting apparatus 6, the control section 14 of the console 7 sends a signal in such a way that, when there is no more need for repeating the radiographing operation, the result of detection on this radiographing operation stored in the radiographic image detecting apparatus 6 is sent to the server 2 or console 7 as appropriate through the communication section 19, and is then deleted.

In the present embodiment, the patient list is generated by the control section 31 of the server 2, with the patient list generating device not being restricted thereto. For example, it is possible to make such arrangements that patient list is generated by the control section 14 of the console 7 and is sent from the console 7 to the server 2. It is then stored in the external storage apparatus 32 of the server 2, and the patient list is sent from the server 2 to each of the consoles 7 where the patient list is shared.

In the present embodiment, after the radiographic image detecting apparatus 6 has been associated with a certain console 7, it is moved between the X-ray rooms 50 for example and is associated with a new console 7. Then, the previous relationship of association between the console 7 and radiographic image detecting apparatus 6 is cancelled. However, it is possible to arrange such a configuration that, when it is moved between the X-ray rooms 50 for example and is associated with a new console 7, the previous relationship of association is kept without being cancelled, and a plurality of overlapped relationships of association is present.

In this case, for example, it is also possible to make such arrangements that the radiographic image obtained by the radiographic image detecting apparatus 6 is associated with the console ID of the console 7 used to operate the radiographic image detecting apparatus 6 at the time of this radiographing operation, whereby this radiographic image is sent to only the console 7 containing this console ID. Thus, this image can be sent to only the console 7 used to operate the radiographic image detecting apparatus 6 at the time of this radiographing operation. After radiographing operation, the image can be verified immediately close to the X-ray room 50 for the convenience of the operator.

In the present embodiment, the radiation image radiographing apparatus 3 is operated by the radiation irradiation operation apparatus 4. It is possible to arrange such a configuration that the radiation image radiographing apparatus 30 is operated by the console 7. This configuration eliminates the need of installing the radiation irradiation operation apparatus 4 and simplifies the system structure.

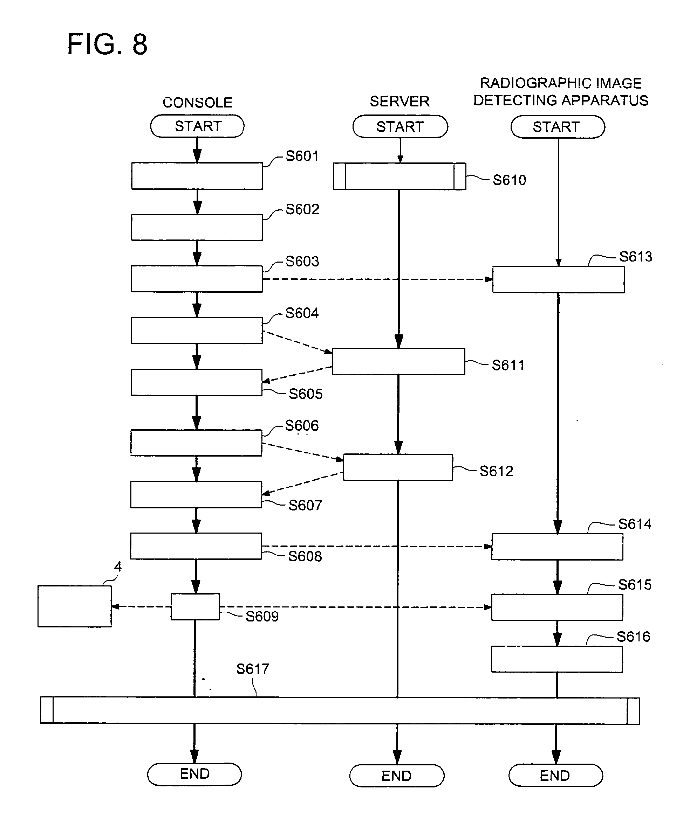

Fig. 8 is a flow chart representing association between the console and radiographic image detecting apparatus and the flow of processing in radiographing operation in the third embodiment. Processing in the Step S601 through Step S609 is executed by the console 7, processing in the Step S610 through Step S612 is executed by the server 2, and processing in the Step S613 through Step S616 is executed by the radiographic image detecting apparatus 6.

Prior to radiographing operation, the identification information such as the detecting apparatus ID of the radiographic image detecting apparatus 6 is inputted by the radiology technician through the input operation section 18 or the like of the console 7 for managing the this X-ray room 50 (Step S601). Processing in the Step S601 is preferably performed at every radiographing operation.

In Step S602, the control section 14 of the console 7 establishes association between the console 7 and radiographic image detecting apparatus 6. Association is established, for example, by the identification information such as the detecting apparatus ID or the like of the radiographic image detecting apparatus 6 being stored into the ROM 16 of the console 7.

In Step S603, the console ID as the identification information of the console 7 is sent as association information to the radiographic image detecting apparatus 6 associated in Step S602. Upon receipt of the console ID, the radiographic image detecting apparatus 6 allows the console ID to be stored in a predetermined area of the ROM 23 of the radiographic image detecting apparatus 6.

Upon completion of association between the console 7 and radiographic image detecting apparatus 6, the radiology technician refers to the patient list stored in the server 2 via the network 8, using the console 7 having been associated (Step S604), and takes action to send the patient list from the server 2 to the console 7a (Step S611) so that the patient list is displayed on the display section 17 of the console 7 (Step S605). The radiology technician selects therefrom the patient 11 to be imaged and, if required, the region to be imaged (selection of the order of radiographing operation) (Step S606).

In response to the transmission of the radiographing order ID corresponding to the order of radiographing operation selected in Step S606, the patient information and radiographing information associated with the radiographing order ID having been sent in Step S606 is sent from the server 2 to the console 7 (Step S612). In this case, the patient information sent from the server 2 includes the planned number of radiographing operations.

The patient information and radiographing information is displayed on the display section 17 of the console 7a as appropriate (Step S607), and patient information including the planned number of radiographing operations and radiographing information are sent to the radiographic image detecting apparatus 6 having been associated (Step S608). It is then stored in a predetermined area of the ROM 23 of the radiographic image detecting apparatus 6 (Step S614). In Step S609, the radiology technician performs radiographic imaging operation while checking the information displayed in Step S607.

In Step S609, the radiographing information having been sent from the server 2 in Step S612 is sent to the radiation irradiation operation apparatus 4, and the radiation irradiation operation apparatus 4 controls the radiation tube 9 of the radiation image radiographing apparatus 3 based on the tube voltage value and radiation dose contained in the received radiographing information. The radiation image radiographing apparatus 3 applies radiation to the patient 11 under these conditions.

In this case, the radiographic image detecting apparatus 6 is located on the examination table 12 and is inserted below the patient 11. It detects the dose of radiation passing through the patient 11 (Step S615), and converts the radiation having been detected, into electric signal, thereby acquiring radiographic image information based on a predetermined result of detection. This radiographic image information is stored in the image memory 25 (Step S616).

In Step S617, image data is transferred for every radiographing operation based on the planned number of radiographing operations contained in the patient information, and a series of radiographing operations then terminate.

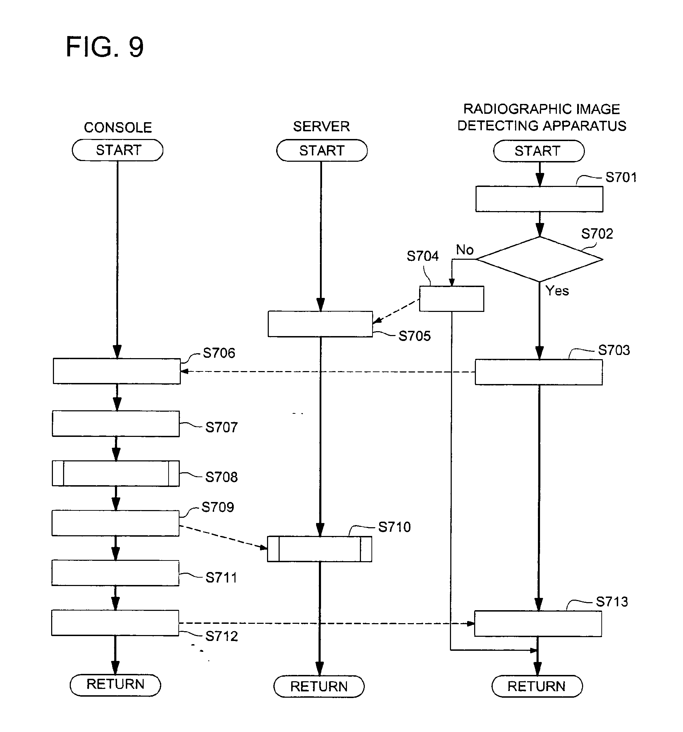

Fig. 9 is a flow chart representing the flow of image data transfer processing executed in Step S617 of Fig. 8.

In Step S701, the planned number of radiographing operations included in the patient information stored in the Step S614 of Fig. 8 is decremented by 1. In Step S702, a decision step is taken to determine whether or not the planned number of radiographing operations having been decremented in Step S701 is zero, namely, whether or not all the radiographing operations of the patient performed in Step S609 of Fig. 8 has terminated. If the planned number of radiographing operations is zero (Yes in Step S702), processing of Step S703 is executed. If the planned number of radiographing operations is not 0 (No in Step S702), the radiographing operation of this patient is regarded as having been performed and the processing of Step S704 is executed.

In Step S703, of the data images stored in the image memory 25, all the images captured in the Step S609 of Fig. 8 are sent to the console 7 containing the console ID stored in the ROM 23. The console 7 containing the console ID stored in the ROM 23 has been associated in Step S602 of Fig. 8, prior to radiographing operation, and is the console last used in the radiographing operation for this patient.

Based on the result of detection having been sent, the control section 14 of the console 7 as a display image generation device generates the so-called thumbnail image whose information size is smaller than that of the original image information (Step S706), and the generated display image is displayed on the display section 1.7 (Step S707). The radiology technician checks the display image to determine whether or not the subject image has been properly detected by the radiographic image detecting apparatus 6, and whether or not there is any need of repeating the radiographing operation (Step S708).

If the subject image has been properly detected and there is no need of repeating the radiographing operation, the control section 14 of the console 7 establishes association between the original image information and patient information, and the information is then sent to the server 2 through the communication section 19 (Step S709). After that, the original image information is processed by the server 2, and is then outputted to such an output device as a printer or monitor as appropriate. This information as a radiographic image is supplied to a doctor for diagnosis (Step S710). In the meantime, the display image is deleted by the control section 14 (Step S711).

The control section 14 of the console 7 sends to the radiographic image detecting apparatus 6 the signal notifying that the radiographic imaging operation is proper, and the deletion instruction signal instructing deletion of the result of detection stored in the radiographic image detecting apparatus 6 (Step S712). Upon receipt of these signals, the control section 21 of the radiographic image detecting apparatus 6 deletes the image data sent from the image memory 25 in Step S703 (Step S713), whereby the processing of image transfer terminates.

In Step S704, the planned number of radiographing operations having been decremented in Step S701 is sent to the server 2 through the console 7. The server 2 ensures that the planned number of radiographing operations included in the patient information captured in Step S609 of Fig. 8 is updated to the planned number of radiographing operations having been sent in Step S704. This arrangement allows the most updated patient information to be stored in the server 2, and makes it possible to get the most updated patient information from any desired console 7, and to perform radiographing operations in a planned manner. When the processing in Step S704 is executed, the processing in Step S703 is not executed. Thus, the process goes back to the processing of Fig. 8 without executing the processing after Step S706 in the console 7 or the processing of Step S710 in server 7.

Thus, according to the radiation image radiographing system 1 of the present embodiment, all the radiographic image information on the patient is sent to the console 7 associated as the one operating the radiographic image detecting apparatus 6 at the time of the last radiographing operation performed for this patient, and the display image appears on the console 7. Thus, independently of the console 7 used to input the patient information and radiographing information and to establish association between the radiographic image detecting apparatus 6 and console 7, the suitability of all the radiographing operations for captured images can be checked using the console 7 located close to the X-ray room 50 wherein the radiology technician is currently performing the radiographing operation. Without having to move far away from the site of radiographing operation, the radiology technician is allowed to check for proper detection of the subject image by comparison immediately and certainly, and to determine the suitability of the radiographic image.

In the present embodiment, a plurality of display images sent to the console 7 are displayed side by side on the display section 17 of the console 7, without the method of display being restricted thereto. For example, it is possible to make such arrangements that a display image is switched over to another as appropriate and is displayed on the display section 17.

In the present embodiment, after the radiographic image information based on the result of detection by the radiographic image detecting apparatus 6 is sent to the console 7 corresponding to the radiographic image detecting apparatus 6, a display image is generated by the control section 14 of the console 7. However, the display image generation device for generating the display image is not restricted to the control section 14 of the console 7.

For example, as in the present embodiment, when the radiographic image detecting apparatus 6 is a cassette type FPD, the display image can be generated by the control section 21 of the radiographic image detecting apparatus 6. In this case, the original image information and display image generated by the control section 21 of the radiographic image detecting apparatus 6 can both be sent to the console 7 or server 2 by a radio communication device or the like. Further, it is possible to make such arrangements that only the display image is sent to the console 7, and the original image information is sent to the server 2. It is also possible to make such arrangements that the original image information is stored in the image memory 25 of the radiographic image detecting apparatus 6, and only the display image is sent to the console 7. In this case, when a signal is sent from the console 7 to the radiographic image detecting apparatus 6 notifying that radiographic imaging conditions are proper, the original image information can be sent to the console 7 or server 2. It is also possible to arrange such a configuration that the radiographic image information obtained by the radiographic image detecting apparatus 6 is directly sent to the server 2, where the display image in addition to the original image information is generated.

In the present embodiment, the patient list is generated by the control section 31 of the server 2, without the patient list generating device being restricted thereto. For example, it is possible to make such arrangements that patient list is generated by the control section 14 of the console 7 and is sent from the console 7 to the server 2. It is then stored in the external storage apparatus 32 of the server 2, and the patient list is sent from the server 2 to each of the consoles 7 where the patient list is shared.

In the present embodiment, the decrement and decision of the planned number of radiographing operations are carried out by the radiographic image detecting apparatus 6. However, they can be performed by either the console 7 or server 2. In this case, the patient information including the planned number of radiographing operations is preferably stored in the console 7 or server 2.

It is possible to arrange such a configuration that, regarding the radiographing data for one patient being sent from the radiographic image detecting apparatus 6 to the console 7, the radiographic image detecting apparatus 6 is provided with an radiographing end button, and the radiology technician presses the radiographing end button, whereby all the image data, instead of, or in addition to, decision on the planned number of radiographing operations, is sent to the last associated console 7. In this case, the need of repeating the radiographing operation can be determined at any desired timing. This allows the patient to be released from the burden of radiographing operation on the temporary basis or at an earlier stage, and makes it possible to meet a sudden change in the bodily conditions.

Further, the detailed structure and operation of the components of the radiation image radiographing system can be modified as appropriate, without departing from the technological spirit and scope of the invention claimed.

## Claims

1. A radiation image radiographing system comprising:
a radiographic image detecting apparatus for detecting a radiation having been applied;
a plurality of consoles for being used to operate the
radiographic image detecting apparatus, the consoles including,
a communication section for communicating with an external apparatus,
the radiation image radiographing system further comprising:
an association device for establishing an association between the radiographic image detecting apparatus and a console among the plurality of consoles, the console operates the radiographic image detecting apparatus during a radiographing operation; and
an image information transmission device for sending radiographic image information obtained based on a detection result of the radiographic image detecting apparatus, to at least the console associated by the association device;
wherein the console has a display section for displaying the radiographic image information.

2. The radiation image radiographing system described in claim 1 further comprising,
a display image generation device for generating a radiographic image for display, based on the detection result of the radiographic image detecting apparatus,
wherein the radiographic image information sent to the console by the image information transmission device is radiographic image information for display generated by the display image generation device.

3. The radiation image radiographing system described in claim 2,
wherein the radiographic image information for display generated by the display image generation device is reduced-size image information whose information amount is smaller than that of original image information.

4. The radiation image radiographing system described in any one of claims 1 through 3,
wherein the radiographic image detecting apparatus is a cassette type flat panel detector (FPD) which detects the radiation having been applied, and converts the radiation into an electric signal to store the electric signal, and then reads the stored electric signal to obtain radiographic image information.

5. The radiation image radiographing system described in claim 1 further comprising:
a patient information inputting device for inputting patient information which identifies a patient as a subject;
a patient information association device for establishing an association between the patient information and the detection result of the radiographic image detecting apparatus; and
an image information transmission device for sending the radiographic image information obtained based on the detection result associated with the patient information, to at least the console associated as the console for operating the radiographic image detecting apparatus during a radiographing operation, the radiographic image detecting apparatus having last radiographed the patient.

6. The radiation image radiographing system described in claim 5 further comprising,
a patient list generating device for generating a patient list based on at least the patient information inputted through the patient information inputting device,
wherein the console has a patient selecting device for selecting a patient from the patient list, and the patient information association device associates the patient information of the patient selected by the patient selecting device for a radiographing operation with the detection result obtained by the radiographing operation.

7. The radiation image radiographing system described in claim 5 or 6 further comprising,
a display image generation device for generating radiographic image information for display based on the detection result of the radiographic image detecting apparatus,
wherein the radiographic image information sent to the console by the image information transmission device is radiographic image information for display generated by the display image generation device.

8. The radiation image radiographing system described in claim 7,
wherein the radiographic image information for display generated by the display image generation device is reduced-size image information whose information amount is smaller than that of original image information.

9. The radiation image radiographing system described in any one of claims 5 through 8,
wherein the radiographic image detecting apparatus is a cassette type flat panel detector (FPD) which detects the radiation having been applied, and converts the radiation into an electric signal to store the electric signal, and then reads the stored electric signal to obtain radiographic image information.
